# EUROPÄISCHE PATENTSCHRIFT

(11) **EP 2 237 757 B1**
(45) Veröffentlichungstag und Bekanntmachung des Hinweises auf die Patenterteilung: **04.03.2015**
(21) Anmeldenummer: 09705460.5
(22) Anmeldetag: 13.01.2009
(51) Int. Cl.: A61F 13/02, A61K 9/70, A61F 13/00, A61F 15/00

(54) **MIKRO- UND/ODER NANOSTRUKTURIERTER PACKSTOFF**
MICRO- AND/OR NANO-STRUCTURED PACKAGING MATERIAL
MATÉRIAU D'EMBALLAGE MICROSTRUCTURÉ ET/OU NANOSTRUCTURÉ

(30) Priorität: 30.01.2008 DE 102008006788
(43) Veröffentlichungstag der Anmeldung: 13.10.2010
(73) Patentinhaber: LTS LOHMANN Therapie-Systeme AG, 56626 Andernach (DE)
(72) Erfinder: MAIER, Stephan, 51371 Leverkusen (DE)
(74) Vertreter: Siemund, Volker
(86) Internationale Anmeldenummer: PCT/EP2009/000125
(87) Internationale Veröffentlichungsnummer: WO 2009/095151

(56) Entgegenhaltungen:
- EP-A- 2 003 178
- DE-A1- 10 056 234
- US-A- 5 613 958
- US-A1- 2003 129 343
- US-A1- 2008 078 500

## Beschreibung

Die Erfindung betrifft einen bahnförmiger Packstoff, der mindestens eine eine Packstoffoberfläche umfassende Schicht aus einem siegelfähigen Werkstoff umfasst, wobei die gesamte Packstoffoberfläche eine Vielzahl von Ausnehmungen und/oder eine Vielzahl nicht ausgenommener Bereiche aufweist, eine Verpackungseinheit mit einem transdermalen therapeutischen System und mit einem derartigen Packstoff sowie ein Verfahren zur Herstellung einer Verpackungseinheit.

Packstoffe mit einer derartigen Oberfläche sind beispielsweise aus der US 2003 129 343 A1 oder der US 5 613 958 A bekannt.

Transdermale therapeutische Systeme verfügen in der Regel über drucksensitive Klebstoffschichten oder drucksensitive Wirk- und Klebstoffschichten zur Fixierung des Systems auf der Haut des Patienten. Während der Lagerung und vor der Applikation auf die Haut sind diese Schichten auf der Klebeseite mit einer abziehbaren Schutzfolie bedeckt. Dies kann jedoch nicht oder nicht völlig verhindern, dass während der Lagerung infolge des "Kalten Flusses" - und im verstärkten Maße bei leicht erhöhten Temperaturen - insbesondere an den seitlichen Randbereichen geringfügige Mengen des haftklebenden Materials austreten kann. Dies kann dazu führen, dass die transdermalen therapeutischen Systeme an der Innenseite der sie umgebenden Verpackung festkleben, was wiederum die Handhabung bei der Entnahme aus der Verpackung erschwert und zur Zerstörung eines transdermalen therapeutischen Systems führen kann. Dadurch entstehen vermeidbare Kosten. Außerdem wird die Akzeptanz dieser Arzneiform bei den Anwendern beeinträchtigt. Bei der Lagerung in Verpackungseinheiten besteht damit die Gefahr, dass aus der klebstoffhaltigen Schicht austretender Klebstoff am Packstoff der Verpackungseinheit anklebt, was eine Entnahme des transdermalen therapeutischen Systems aus der Verpackungseinheit erschwert.

Um ein Ankleben des transdermalen therapeutischen Systems an der Innenseite der Verpackung zu verhindern, kann beispielsweise eine Noppung als Abstandshalter zur oberen Packstoffinnenseite in den über die klebstoffhaltige Schicht überstehenden Rand einer Schutzfolie eingestanzt werden. Da das Fördern der Produkte meist mittels Klemmbacken erfolgt, kann hierbei die Noppung verformt und damit als Abstandshalter unbrauchbar werden. Dieses Verfahren verhindert somit kaum eine Verklebung des transdermalen therapeutischen Systems mit der Packstoffinnenfläche.

Weiterhin kann zusätzlich zu einer an der klebstoffhaltigen Schicht anhaftenden, überstehenden, silikon- oder fluorpolymerbeschichteten Schutzfolie eine überstehende, silikon- oder fluorpolymerbeschichtete Deckfolie auf eine Trägerfolie des transdermalen therapeutischen Systems aufgelegt werden, um ein Ankleben von am Pflasterrand austretendem Klebstoff an der der Trägerfolie zugewandten Packstoffinnenfläche zu verhindern. Dies erfordert ein technisch aufwendiges Einbinden der Deckfolie in den Herstellungsprozess und die Deckfolie muss bei der Formulierung des transdermalen therapeutischen Systems berücksichtigt werden. Außerdem muss die Deckfolie bei der Anwendung des transdermalen therapeutischen Systems zusätzlich entsorgt werden.

Eine Reihe von transdermalen therapeutischen Systemen oder Wirkstoffpflastern weisen wegen ihrer Zusammensetzung oder Inhaltsstoffe eine spezielle Empfindlichkeit auf und neigen dazu, am Packstoff anzukleben.

Der vorliegenden Erfindung liegt daher die Problemstellung zugrunde, das Ankleben eines Klebstoffs an der Packstoffinnenfläche wirksam zu verhindern.

Diese Problemstellung wird mit den Merkmalen des Hauptanspruches dadurch gelöst, dass der Abstand zweier benachbarter Ausnehmungen kleiner sind als ein Mikrometer und/oder der Abstand zweier benachbarter nicht ausgenommener Bereiche kleiner sind als ein Mikrometer und dass die Tiefe der Ausnehmungen minimal 1,2 Nanometer und maximal 95 % der Packstoffdicke ist.

Bei der Herstellung der Verpackungseinheit wird zumindest eine Oberfläche eines Packstoffs mit einer Vielzahl von Ausnehmungen versehen und/oder mit Ausnehmungen versehen, die eine Vielzahl von nicht ausgenommenen Bereichen umgeben. Die Ausnehmungen werden derart erzeugt, dass der Abstand zweier benachbarter Ausnehmungen und/oder der Abstand zweier benachbarter nicht ausgenommener Bereiche kleiner ist als das Fünffache der Packstoffdicke und die Tiefe der Ausnehmungen minimal 1,2 Nanometer und maximal 95 % der Packstoffdicke ist. Diese Oberfläche des Packstoffs wird auf die der klebstoffhaltigen Schicht abgewandten Seite des transdermalen therapeutischen Systems aufgelegt. Dieser erste Packstoff wird mit einem zweiten Packstoff, der auf der klebstoffhaltigen Schicht zugewandten Seite des transdermalen therapeutischen Systems angeordnet ist, mittels Heißsiegeln verbunden. Hierbei wird eine feuchtigkeits- gas- und aromadichte Fuge erzeugt.

Weitere Einzelheiten der Erfindung ergeben sich aus den Unteransprüchen und der nachfolgenden Beschreibung schematisch dargestellter Ausführungsformen.
- Figur 1:: Verpackungseinheit mit transdermalem therapeutischen System;
- Figur 2:: Detail eines Packstoffs;
- Figur 3:: Detail eines Klebertropfens auf einem Packstoff;
- Figur 4:: Spitz ausgeformte Strukturen des Packstoffs;
- Figur 5:: Mikro- und nanostrukturierter Packstoff;
- Figur 6:: Variante eines mikro- und nanostrukturierten Packstoffs;
- Figur 7:: Detail einer Fügenaht;
- Figur 8:: Verpackungseinheit mit Transdermalem therapeutischen System ohne Schutzfolie.

Die Figur 1 zeigt eine Verpackungseinheit (10) mit einem transdermalen therapeutischen System (21). Die Verpackungseinheit (10) umfasst zwei Packstoffe (31, 41), die das transdermale therapeutische System (21) feuchtigkeits-, gas- und aromadicht umschließen.

Das transdermale therapeutische System (21) ist z.B. ein wirkstoffhaltiges Pflaster (21) mit einer Klebematrix. Der Wirkstoff (23) und der Klebstoff (24) sind in diesem Ausführungsbeispiel in einer gemeinsamen Schicht (22) an einer Trägerfolie (27) angeordnet. Der Wirk- (23) und der Klebstoff (24) können jedoch auch in getrennten Schichten angeordnet sein, wobei zumindest die der Trägerfolie (27) abgewandte Schicht (22) klebstoffhaltig ist. In einer Draufsicht auf das transdermale therapeutische System (21) haben die Trägerfolie (27) und die Wirk- und Klebstoffschicht (22) beispielsweise die gleiche Größe. Unterhalb der Wirk- und Klebstoffschicht (22) liegt im Ausführungsbeispiel der Figur 1 eine Schutzfolie (28), die z.B. an der Wirk- und Klebstoffschicht (22) anhaftet. Diese Schutzfolie (28) steht an den Rändern (29) über die Wirk- und Klebstoffschicht (22) über.

Der Klebstoff (24), der bei der Applikation des transdermalen therapeutischen Systems (21) das Haften auf der Haut des Patienten sicherstellt, ist beispielsweise drucksensitiv. Schon bei geringem Druck oder leicht erhöhter Temperatur kann sich bei diesem Klebstoff (24) ein sogenannter "Kalter Fluss" ausbilden. Der Klebstoff (24) quillt dann über die eigentliche Pflasterkontur, also über die Fläche der Trägerfolie (27) hinaus. Der Klebstoff (24) besteht im Wesentlichen z.B. aus einem matrixbildenden Haftkleber. Hierfür können beispielsweise Polyacrylate, Silikone, Polyisobutylene, Kautschuk, kautschukähnliche synthetische Homo-, Co- oder Blockpolymere, Butylkautschuk, Styrol/Isopren-Copolymerisate, Polyurethane, Copolymere des Ethylens, Polysiloxane oder Styrol/Butadien Copolymerisate einzeln und/oder in Kombination verwendet werden. Der Klebstoff (24) kann aber auch weitere Stoffe, wie z.B. physiologisch wirksame Stoffe, Farbstoffe, Weichmacher, Klebrigmacher, Permeationsförderer, etc., enthalten. Die Oberflächenspannung des Klebstoffs (24) gegen seine Dampfphase beträgt z.B. zwischen 30 und 50 Millinewton pro Meter.

Die beiden Packstoffe (31, 41) bestehen z.B. aus einem einschichtigen folienartigen siegelfähigen Werkstoff oder aus einem mehrschichtigem Packstofflaminat. Beim mehrschichtigen Packstofflaminat besteht zumindest eine eine Packstoffoberfläche umfassende Schicht aus einem siegelfähigen Werkstoff. Die Dicke der siegelfähigen Packstoffschicht oder des einschichtigen Packstoffs beträgt beispielsweise ein Zehntel Millimeter, sie kann aber auch dünner sein. Diese z.B. aromadicht, wasserdicht und/oder sauerstoffdicht ausgeführte Folie besteht beispielsweise aus einem thermoplastischen Werkstoff, z.B. aus Polyester, Polyethylen, Polypropylen, Polyamid, Acrylnitril-Methyl-Acrylat-Copolymerisate, Ethylen-Vinylacetat-Copolymere, Ethylen-Copolymerisate, Ionomere, etc. oder deren Mischungen.

Bei der Siegelung - sowohl bei einer Heiß- als auch bei einer Kaltsiegelung - wird eine nahezu homogene Verbindung der Siegelschichten des oberen und unteren Siegellaminats erreicht. Für die Heißsiegelung werden Heißsiegeldispersionen, Heißsiegellacke, Schmelzklebestoffe sowie Filme aus thermoplastischen Elastomeren und Extrusionsbeschichtungen eingesetzt. Eine Kaltsiegelung erfolgt beispielsweise unter Einsatz von Feuchtigkeit, Lösemitteln, oder sonstigen Kontakthilfsmitteln, z.B. Kaltsiegelmassen.

Um die geforderte Dichtigkeit gegen Verluste von teilweise flüchtigen Wirk- oder sonstigen Inhaltsstoffen zu bewirken, werden die üblichen, für die Verpackung von transdermalen therapeutischen Systemen verwendeten Packstoffe zusätzlich mit einer Barriereschicht, einer Sperrschicht, ausgestattet. Diese stellt in der Regel die auf die Siegelschicht folgende nächstinnere Schicht dar. Die Barriereschicht kann z.B. aus einer durchgehenden Metallschicht, beispielsweise einer Aluminiumschicht, bestehen, jedoch kann grundsätzlich auch ein diffusionsdichtes Kunststoffmaterial, z.B. Polyethylenterephthalat in Frage kommen. Zusätzlich können die Verpackungslaminate mit weiteren Schichten versehen sein, die in der Regel außenseitig angebracht sind, und die z.B. aus Papier oder Kunststofffolien bestehen können. Sie dienen beispielsweise der verbesserten Bedruckbarkeit, der Sicherheit vor unerwünschten Zerreißen (Kindersicherheit) oder einer ästhetisch ansprechenden Gestaltung. Die Dicke der Packstofflaminate beträgt beispielsweise ein Zehntel Millimeter. Sie kann aber auch dicker sein.

Die Packstoffe (31, 41) haben einander zugewandte beispielsweise siegelfähige Oberflächen (32, 42) und sind im Ausführungsbeispiel der Figur 1 thermisch durch Heißsiegeln z.B. mittels vier das transdermale System (21) umgebende Fügenähten (51) verschmolzen. Zumindest die nach innen gerichtete Oberfläche (32) des oberen Packstoffs (31) hat keine Silikonoder Fluorpolymerbeschichtung.

Eine zum Beispiel als Siegelmedium dienende thermoplastische Werkstoffschicht auf der Innenseite des Packstoffs hat bei glatter Oberfläche beispielsweise eine Oberflächenspannung, die gleich der Oberflächenspannung des eingesetzten Klebstoffs (24) ist oder von dessen Wert z.B. um maximal 20 % abweicht. Der Klebstoff (24) und eine glatte Oberfläche des Siegelmediums haben damit miteinander eine starke Neigung zum Verkleben. Das Verkleben erzeugt eine feste Verbindung, die sich nur unter großem Kraftaufwand, z.B. mit einer spezifischen Kraft größer als 5 Newton pro 25 Millimeter Packstoffbreite, lösen lässt. Demgegenüber bedeutet Anhaften, dass sich Packstoff (31; 41) und transdermales therapeutisches System (21) mit geringem Kraftaufwand, z.B. kleiner als der genannte Kraftwert, rückstandsfrei von Hand voneinander trennen lassen.

Die in der Figur 1 nach innen gerichtete Oberfläche (32) des oberen Packstoffs (31) weist Ausnehmungen (33) und nicht ausgenommene Bereiche (34) auf. Ein Beispiel einer derartigen Struktur zeigt die Figur 2. Die hier als Ausschnitt dargestellte Struktur hat zylinderförmige Ausnehmungen (33), die von einem gitterförmigen nicht ausgenommenen Bereich (34) umgeben sind. Die Tiefe der Ausnehmungen (33) beträgt z.B. 100 Nanometer. Sie kann zwischen 1,2 Nanometer und 95 % der Packstoffdicke betragen. Der Durchmesser der hier dargestellten Ausnehmungen (33) beträgt z.B. 50 Nanometer. Er kann beispielsweise bis zum Fünffachen der Packstoffdicke betragen.

Die Gitterstäbe (35) der nicht ausgenommenen Bereiche (34) haben hier beispielsweise eine Dicke von maximal 50 Nanometern, so dass in diesem Ausführungsbeispiel zwei einander benachbarte Ausnehmungen (33) einen Abstand von 50 Nanometern haben. Dieser Abstand kann bis zum Fünffachen der Packstoffdicke betragen. Die Stirnfläche (36) ist beispielsweise eine Planfläche.

Im Ausführungsbeispiel ist die Vielzahl von Ausnehmungen (33) und der nicht ausgenommene Bereiche (34) regelmäßig angeordnet. Die Struktur kann aber auch unregelmäßig angeordnet sein, die Tiefen der Ausnehmungen (33) können unterschiedlich sein. Die Bodenflächen (37) können plan, konkav, konvex, etc. ausgebildet sein.

Bei einer Struktur mit einer Vielzahl von nicht ausgenommenen Bereichen (34) können diese beispielsweise als Stäbe mit quadratischem, runden, rechteckigen, dreieckigen, etc. Querschnitt ausgebildet sein. Die Stirnflächen (36) können dann plan oder konvex ausgebildet sein. Die nicht ausgenommenen Bereiche (34) können kegel- oder pyramidenförmig, pilzförmig, etc. ausgebildet sein.

Durch die Strukturierung der Oberfläche (32) ergibt sich bei einem durch z.B. kalten Fluss hervorgerufenen Kontakt der Packstoffinnenfläche mit einem Klebstoff eine mit dem Klebstoff in Kontakt tretende resultierende wirksame Oberfläche, deren Eigenschaften sich von den Eigenschaften des Grundwerkstoffs unterscheiden. So zeigt eine entsprechend strukturierte Oberfläche (32) z.B. gegenüber einem aufgebrachten Klebstofftropfen eine deutlich geringere Oberflächenenergie als der glatte Grundwerkstoff. Hierdurch können z.B. adhäsive Bindungen zwischen dem Klebstoff (24) und der Packstoffinnenseite nur im geringen Maße ausgebildet werden. Ein Verkleben des Packstoffs (31) mit dem transdermalen therapeutischen System (21) wird somit wirksam verhindert.

Der in der Figur 1 untenliegende Packstoff (41) kann eine nach innen gerichtete glatte Oberfläche (42) aufweisen. Die innere Oberfläche (42) des unteren Packstoffs (41) kann aber auch eine Struktur aufweisen, wie sie im Zusammenhang mit der inneren Oberfläche (32) des oberen Packstoffs (31) beschrieben ist. Bei einer Ausführung der Verpackungseinheit als Dreirandsiegelbeutel kann der untere Packstoff (41) Teil des oberen Packstoffs (31) sein.

Die außenliegenden Oberflächen (38, 48) der Packstoffe (31, 41) können glatt oder strukturiert sein.

Die Herstellung der Verpackungseinheit (10) mit dem transdermalen therapeutischen System (21) erfolgt beispielsweise in einem mehrstufigen verketteten Prozess. So wird z.B. zunächst die wirk- und klebstoffhaltige Schicht (22) auf die Trägerfolie (27) beschichtet. Hierbei ist beispielsweise die Trägerfolie (27) die Transportfolie, mit deren Hilfe das Zwischenprodukt durch die Herstellungsvorrichtung gefördert wird. Nach dem Trocknen oder Erkalten der wirk- und klebstoffhaltigen Schicht (22) wird diese mit der Schutzfolie (28) vollflächig abgedeckt. Alternativ hierzu kann auch im ersten Schritt die Schutzfolie (28) mit der wirk- und klebstoffhaltigen Schicht (22) beschichtet werden. Nach dem Trocknen oder Erkalten der wirk- und klebstoffhaltigen Schicht (22) wird diese dann mittels der Trägerfolie (27) vollflächig abgedeckt. Das so vorbereitete Laminat wird nun in Längsrichtung geschnitten und anschließend einer Stanz- und Verpackungsstation zugeführt.

In der Stanz- und Verpackungsstation wird das einzelnen transdermale therapeutische System (21) aus dem bahnförmigen Laminat ausgestanzt und anschließend entweder mit der Schutzfolie (28) beispielsweise auf die innere Oberfläche (42) einer unteren Packstoffbahn aufgesetzt, mit der oberen Packstoffbahn abgedeckt und rundum versiegelt oder aber direkt zwischen eine obere und eine untere Packstoffbahn gespendet und rundum versiegelt. Die Packstoffbahnen werden jeweils von einer Rolle abgewickelt und beispielsweise kontinuierlich mittels Klemmbacken- oder Zangenvorschub gefördert.

An der oberen Packstoffbahn wird vor dem Einsatz die innere Oberfläche (32) vorbereitet. Dies kann bereits außerhalb der Verpackungsstation erfolgen. Die aufzubringende Grundstruktur lässt sich beispielweise durch holografische Aufzeichnungsmethode erzeugen. Dies wird z.B. durch die Technik der Zweistrahlinterferenz auf der Basis kohärenter optischer Systeme oder von Elektronenstrahlsystemen umgesetzt. Hierbei wird beispielsweise eine mit Fotolack beschichtete Glasplatte in ein von Laserstrahlen erzeugtes Interferenzmuster eingebracht. Durch die Belichtung entsteht auf dem Lack ein Muster, dessen Abstände z.B. im Nanometerbereich liegen. Mit Hilfe der durch Metallisierung leitfähig gemachten Glasplatten können z.B. durch Elektroforming oder durch Galvanische Replikation mittels Nickelabscheidung Kopien der strukturierten Oberfläche hergestellt werden. Diese Kopien sind in Form von Platten oder dünnen Nickelfolien herstellbar. Die Formen können dann mittels Spritzguss, thermoplastischer Abformung oder mittels Walzen auf die Innenseite der Packstofffolie übertragen werden.

Beispielsweise wird die Struktur ganzflächig auf die z.B. siegelbare Oberfläche der Packstoffbahn aufgebracht.

Der so vorbereitete bahnförmige obere Packstoff (31) wird beispielsweise so auf den bahnförmigen unteren Packstoff (41) und das transdermale therapeutische System (21) aufgelegt, dass die strukturierte innere Oberfläche (32) zum transdermalen therapeutischen System (21) zeigt. Anschließend werden der untere (41) und der obere Packstoff (31) z.B. an allen vier Rändern (52) miteinander versiegelt, beispielsweise mittels Heißsiegeln.

Beim Heißsiegeln werden die beiden chemisch gleichartigen, strukturell z.B. unterschiedlichen Oberflächen (32, 42) unter Erhitzung miteinander verbunden. Hierbei wird im Bereich der Heißsiegelnähte (51) die Strukturierung der inneren Oberfläche (32) des oberen Packstoffs (31) geschmolzen, so dass eine aroma-, gas- und feuchtigkeitsdichte Fuge entsteht. In der Figur 7 ist dies für ein Ausführungsbeispiel dargestellt, dessen oberer (31) und unterer Packstoff (41) jeweils einander zugewandte strukturierte Oberflächen (32, 42) aufweisen.

Beispielsweise nach dem Siegeln werden die bahnförmigen Packstoffe (31, 41) durchtrennt. Es entstehen Verpackungseinheiten (10), die jeweils z.B. ein transdermales therapeutisches System (21) umfassen.

Beim Transport oder bei der Lagerung können die Verpackungseinheiten (10) auf Druck belastet oder leicht erhöhten Temperaturen ausgesetzt werden. Dadurch ist es möglich, dass der Klebstoff (24) seitlich über den Rand der Trägerfolie (27) aus der Matrix heraustritt. Ein Verkleben mit der unteren Packstoffinnenfläche (42) wird im Ausführungsbeispiel der Figur 1 durch die überstehenden Ränder (29) der Schutzfolie (28) verhindert. Am oberen Packstoff (31) legt sich der Klebstoff (24) an die nicht ausgenommenen Bereiche (34) an, vgl. die Schnittdarstellung in der Figur 3. Aufgrund der kleinen Kontaktfläche - sie entspricht in diesem Ausführungsbeispiel jeweils einem Abschnitt der Stirnfläche (36) eines nicht ausgenommenen Bereichs (34) - benetzt der Klebstoff (24) die Packstoffinnenseite (32) kaum und zieht sich beispielsweise tropfen- oder linsenförmig zusammen. Beispielsweise bildet sich zwischen dem in der Figur 3 dargestellten Klebstofftropfen (71) und dem Packstoff (31) ein Kontaktwinkel (61) von 160 Grad. Der Klebstofftropfen (71) verhält sich hierbei genauso wie eine Klebstoffschicht, die z.B. flächenförmig auf die strukturierte Oberfläche (32) des Packstoffs (31) aufgebracht wird. Der Klebstofftropfen (71) liegt nur lose am oberen Packstoff (31) an oder haftet leicht an diesem an. Beim Herausnehmen des Pflasters (21) aus der Verpackungseinheit (10) wird er rückstandsfrei entfernt.

Aufgrund der Strukturierung der Oberfläche (32) werden z.B. die physikalischen Eigenschaften der Bindung des Packstoffs (31) mit dem Klebstoff (24) beeinflusst. So ergibt sich eine gegenüber einem vollflächigem Klebstoffauftrag deutlich verringerte resultierende wirksame Oberfläche, wodurch auch die wirksame Oberflächenergie der Packstoffoberfläche (32) gegenüber dem unstrukturierten Werkstoff verringert ist. Hierdurch entsteht zwischen dem Packstoff (31) und dem Klebstoff (24) nur eine schwache adhäsive Bindung. Ein Verkleben der beiden Werkstoffe wird verhindert.

In der Figur 4 ist ein Klebstofftropfen (72) dargestellt, der sich über mehrere - hier beispielsweise pyramidenförmig ausgebildeten - nicht ausgenommene Bereiche (34) erstreckt. Bereichsweise ist er in die Ausnehmungen (33) eingedrungen. Oberhalb des Klebstofftropfens (72) hat sich in den Ausnehmungen (33) ein Luftpolster (79) gebildet, das ein weiteres Eindringen des Klebstofftropfens (72) in die Ausnehmung (33) verhindert. Die Tiefe der Ausnehmung (33) oberhalb des Klebstofftropfens (72) ist größer als die Reichweite der chemischen und der physikalischen adhäsiven Kräfte zwischen den Werkstoffen. Die Reichweite der letztgenannten Kräfte liegt beispielsweise zwischen 0,2 Nanometern und einem Nanometer. Die Tiefe der Ausnehmungen (33) beträgt mindestens 1,2 Nanometer.

Auf dieser aus Luftpolstern (79) und nicht ausgenommenen Bereichen (34) zusammengesetzten wirksamen Oberfläche (62)-derartige Oberflächen werden z.B. als Kompositoberflächen bezeichnet - liegt der Klebstofftropfen (72) nur mit geringer Haftung an. Beispielsweise bildet er in den Ausnehmungen (33) mit den Flanken (39) der nicht ausgenommenen Bereiche (34) einen Kontaktwinkel von z.B. 160 Grad.

In der Figur 5 ist ein Packstoff (31) mit einer Mikro- (67) und mit einer Nanostruktur (66) dargestellt, auf dem mehrere Klebstofftropfen (73 - 75) aufliegen. Die Mikrostruktur (67) hat in dem dargestellten Schnitt beispielsweise die Gestalt einer Sinuskurve. Der Abstand der einzelnen Maxima (69)- dieser ist z.B. zwischen einem Mikrometer und dem Fünffachen der Packstoffdicke - ist in diesem Ausführungsbeispiel so groß, dass die Klebstofftropfen (73 - 75) der Kontur folgen können. Entlang der Mikrostruktur (67) ist eine Nanostruktur (66) in den Packstoff eingebracht. Der Abstand der einzelnen Ausnehmungen (33) der Nanostruktur (66) ist geringer als ein Mikrometer. Auch der Abstand der in der Schnittdarstellung der Figur 5 dargestellten nicht ausgenommenen Bereiche (34) der Nanostruktur (66) ist kleiner als ein Mikrometer. Die Nanostruktur (66) ist beispielsweise so aufgebaut, wie im Zusammenhang mit den Figuren 1 bis 3 beschrieben. Der Packstoff kann auch nur mit einer Mikrostruktur (67) oder nur mit einer Nanostruktur (66) versehen sein.

Die Kompositoberfläche mit den Luftpolster (79) in den Ausnehmungen (33) verhindert ein zu starkes Anhaften und ein Ankleben der Klebstofftropfen (72, 73, 74) am Packstoff (31).

Die Figur 6 zeigt einen Packstoff (31) mit einer Mikro- (67) und einer Nanostruktur (66), bei der die Wellenlänge der Mikrostruktur (67) kürzer ausgebildet ist als in der Darstellung der Figur 5. Der Klebstofftropfen (76) folgt nicht der Kontur der Mikrostruktur (67) sondern liegt nur an deren Maxima (69) auf. Sollte dennoch, z.B. aufgrund von Temperatur- oder Druckeinflüssen ein Klebstofftropfen (76) der Kontur der Mikrostruktur (67) folgen, verhindert die überlagerte Nanostruktur (66) ein Ankleben des Tropfens (76).

In der Figur 8 ist eine Verpackungseinheit (10) dargestellt, bei der das transdermale therapeutische System (21) ohne Schutzfolie (28) ausgeführt ist. Zur Herstellung dieser Ausführungsform kann beispielsweise die strukturierte Packstoffunterbahn (41) entweder wie eine Schutzfolie direkt mit der wirk- und klebstoffhaltigen Schicht (22) beschichtet werden oder es wird eine Trägerfolie (27) mit der wirk- und klebstoffhaltigen Schicht (22) beschichtet. Nach dem Trocknen und/oder dem Erkalten wird diese Schicht (22) direkt mit der strukturierten unteren Packstoffbahn (41) abgedeckt. Die Packstoffunterbahn (41) übernimmt in dieser Ausführungsform zusätzlich alle Funktionen einer Schutzfolie, sowohl bei der Herstellung als auch in der fertigen Verpackungseinheit. In diesem Ausführungsbeispiel sind die dem transdermalen therapeutischen System (21) zugewandten Oberflächen (32, 42) des unteren Packstoffs (41) und des oberen Packstoffs (31) strukturiert, wie dies z.B. in der Figur 2 dargestellt ist. Die beiden einander zugewandten Oberflächen (32, 42) können aber auch so ausgeführt sein, wie in den Figuren 4 - 6 dargestellt. Beim Heißsiegeln werden die Mikro- (67) und/oder Nanostrukturen (66) beider Packstoffe (31, 41) im Verbindungsbereich aufgelöst, vgl. Figur 7. Beim Herausnehmen des transdermalen therapeutischen Systems (21) aus der Verpackungseinheit (10) ist dieses somit weder mit dem unteren (41) noch mit dem oberen Packstoff (31) fest verklebt. Es haftet lediglich schwach an der unteren und/oder oberen Packstoffinnenfläche an und kann leicht und rückstandsfrei abgezogen werden.

Weiterhin beeinträchtigt die beschriebene Mikro- und/oder Nanostruktur der Packstoffe (31, 41) nicht den optischen Eindruck der Verpackungseinheit (10), beispielsweise bei einer Ausführung der Verpackungseinheit (10) mit transparenten Parkstoffen (31, 41).

Auch Kombinationen der beschriebenen Ausführungsbeispiele sind denkbar.

### Bezugszeichenliste:

- 10: Verpackungseinheit

- 21: Transdermales therapeutisches System, Pflaster
- 22: Klebstoffhaltige Schicht, Wirk- und Klebstoffschicht
- 23: Wirkstoff
- 24: Klebstoff

- 27: Trägerfolie
- 28: Schutzfolie
- 29: Ränder

- 31: oberer Packstoff, erster Packstoff
- 32: innere Oberfläche von (31), Packstoffoberfläche
- 33: Ausnehmungen
- 34: nicht ausgenommene Bereiche
- 35: Gitterstäbe
- 36: Stirnflächen
- 37: Bodenflächen
- 38: außenliegende Oberfläche
- 39: Flanken

- 41: unterer Packstoff, zweiter Packstoff
- 42: innere Oberfläche von (41)

- 48: außenliegende Oberfläche

- 51: Fügenähte, Heißsiegelnähte
- 52: Ränder

- 61: Kontaktwinkel
- 62: wirksame Oberfläche

- 66: Nanostruktur
- 67: Mikrostruktur

- 69: Maxima von (67)

- 71 - 76: Klebstofftropfen

- 79: Luftpolster

## Patentansprüche

1. Bahnförmige Packstoff (31), der mindestens eine eine Packstoffoberfläche (32) umfassende Schicht aus einem siegelfähigen Werkstoff umfasst,
wobei die gesamte Packstoffoberfläche (32) eine Vielzahl von Ausnehmungen (33) und eine Vielzahl nicht ausgenommener Bereiche (34) aufweist,
**dadurch gekennzeichnet,**
- **dass** der Abstand zweier benachbarter Ausnehmungen (33) kleiner sind als ein Mikrometer und/oder der Abstand zweier benachbarter nicht ausgenommener Bereiche (34) kleiner sind als ein Mikrometer und
- **dass** die Tiefe der Ausnehmungen (33) minimal 1,2 Nanometer und maximal 95 % der Packstoffdicke ist.

2. Packstoff (31) nach Anspruch 1,
**dadurch gekennzeichnet,**
**dass** die durch die Ausnehmungen (33) gekennzeichnete Strukturierung vollflächig in dieser Packstoffoberfläche (32) eingebracht ist.

3. Verpackungseinheit (10) mit einem transdermalen therapeutischen System (21), das zwischen einem ersten Packstoff (31) und einem mit diesem aroma-, gas- und feuchtigkeitsdicht versiegelten zweiten Packstoff (41) angeordnet ist, wobei die Packstoffe (31, 41) nach Anspruch 1 ausgebildet sind.

4. Verpackungseinheit (10) nach Anspruch 3,
**dadurch gekennzeichnet,**
**dass** der Klebstoff (24) drucksensitiv ist.

5. Verpackungseinheit (10) nach Anspruch 3,
**dadurch gekennzeichnet,**
**dass** das transdermale therapeutische System (21) eine auf allen Seiten überstehende Schutzfolie (28) aufweist.

6. Verpackungseinheit (10) nach Anspruch 3,
**dadurch gekennzeichnet,**
**dass** beide einander zugewandten Oberflächen (32, 42) der Packstoffe (31, 41) eine Vielzahl von Ausnehmungen (33) und/oder eine Vielzahl von nicht ausgenommenen Bereichen (34) aufweist.

7. Verpackungseinheit (10) nach Anspruch 3 oder 6,
**dadurch gekennzeichnet,**
**dass** der zweite Packstoff (41) Teil des ersten Packstoffs (31) ist.

8. Verfahren zur Herstellung einer Verpackungseinheit (10) mit einem eine klebstoffhaltige Schicht (22) enthaltenden transdermalen therapeutischen System (21),
- wobei zumindest die gesamte Oberfläche (32) eines ersten Packstoffs (31) mit einer Vielzahl von Ausnehmungen (33) und mit Ausnehmungen, die eine Vielzahl von nicht ausgenommenen Bereichen (34) umgeben, versehen wird,
- wobei die Ausnehmungen (33) derart erzeugt werden, dass der Abstand zweier benachbarter Ausnehmungen (33) und/oder der Abstand zweier benachbarter nicht ausgenommener Bereiche (34) kleiner ist als das Fünffache der Packstoffdicke und die Tiefe der Ausnehmungen (33) minimal 1,2 Nanometer und maximal 95 % der Packstoffdicke ist,
- wobei diese Oberfläche (32) des Packstoffs (31) auf die der klebstoffhaltigen Schicht (22) abgewandten Seite des transdermalen therapeutischen Systems (21) aufgelegt wird,
- wobei dieser erste Packstoff (31) mit einem zweiten Packstoff (41), der auf der Wirk- und Klebstoffschicht (22) zugewandten Seite des transdermalen therapeutischen Systems (21) angeordnet ist, mittels Siegeln zur Erzeugung feuchtigkeits-, gas- und aromadichte Fügenähte (51) verbunden wird.

## Claims

1. Web-form packaging material (31) which comprises at least one layer, comprising a packaging material surface (32), of a sealable material, where the entire packaging material surface (32) has a multiplicity of recesses (33) and a multiplicity of non-recessed regions (34), **characterized**
- **in that** the distance between two adjacent recesses (33) is less than one micrometer and/or the distance between two adjacent non-recessed regions (34) is less than one micrometer, and
- **in that** the depth of the recesses (33) is not less than 1.2 nanometers and not more than 95% of the packaging material thickness.

2. Packaging material (31) according to Claim 1, **characterized in that** the structuring marked by the recesses (33) is made over the full area in this packaging material surface (32).

3. Packaging unit (10) with a transdermal therapeutic system (21) which is arranged between a first packaging material (31) and a second packaging material (41) which is sealed to the first packaging material in an aroma-proof, gas-proof, and moisture-proof manner, the packaging materials (31, 41) being configured according to Claim 1.

4. Packaging unit (10) according to Claim 3, **characterized in that** the adhesive (24) is pressure-sensitive.

5. Packaging unit (10) according to Claim 3, **characterized in that** the transdermal therapeutic system (21) has a protective film (28) which juts out on all sides.

6. Packaging unit (10) according to Claim 3, **characterized in that** both mutually facing surfaces (32, 42) of the packaging materials (31, 41) have a multiplicity of recesses (33) and/or a multiplicity of non-recessed regions (34).

7. Packaging unit (10) according to Claim 3 or 6, **characterized in that** the second packaging material (41) is part of the first packaging material (31).

8. Process for producing a packaging unit (10) with a transdermal therapeutic system (21) comprising an adhesive-containing layer (22),
- where at least the entire surface (32) of a first packaging material (31) is provided with a multiplicity of recesses (33) and with recesses which surround a multiplicity of non-recessed regions (34),
- where the recesses (33) are produced such that the distance between two adjacent recesses (33) and/or the distance between two adjacent non-recessed regions (34) is less than five times the packaging material thickness, and the depth of the recesses (33) is not less than 1.2 nanometers and not more than 95% of the packaging material thickness,
- where this surface (32) of the packaging material (31) is placed onto the side of the transdermal therapeutic system (21) that faces away from the adhesive-containing layer (22),
- where this first packaging material (31) is joined to a second packaging material (41), which is arranged on the side of the transdermal therapeutic system (21) that faces the active ingredient and adhesive layer (22), by means of sealing, to produce join seams (51) which are moisture-proof, gas-proof, and aroma-proof.

## Revendications

1. Matériau d'emballage (31), qui comprend au moins une couche en une matière soudable qui comprend au moins une surface de matériau d'emballage (32), dans lequel toute la surface de matériau d'emballage (32) présente une multiplicité d'évidements (33) et une multiplicité de zones non évidées (34), **caractérisé en ce que**
- la distance de deux évidements voisins (33) est plus petite qu'un micromètre et/ou la distance de deux zones non évidées voisines (34) est plus petite qu'un micromètre, et
- la profondeur des évidements (33) vaut au minimum 1,2 nanomètres et au maximum 95 % de l'épaisseur du matériau d'emballage.

2. Matériau d'emballage (31) selon la revendication 1, **caractérisé en ce que** la structuration **caractérisée par** les évidements (33) est formée sur toute la surface dans cette surface de matériau d'emballage (32).

3. Unité d'emballage (10) avec un système thérapeutique transdermique (21), qui est disposé entre un premier matériau d'emballage (31) et un deuxième matériau d'emballage (41) soudé à celui-ci d'une manière étanche aux arômes, aux gaz et à l'humidité, dans laquelle les matériaux d'emballage (31, 41) sont réalisés selon la revendication 1.

4. Unité d'emballage (10) selon la revendication 3, **caractérisée en ce que** l'adhésif (24) est sensible à la pression.

5. Unité d'emballage (10) selon la revendication 3, **caractérisée en ce que** le système thérapeutique transdermique (21) présente une feuille de protection (28) dépassant sur tous les côtés.

6. Unité d'emballage (10) selon la revendication 3, **caractérisée en ce que** les deux surfaces tournées l'une vers l'autre (32, 42) des matériaux d'emballage (31, 41) présentent une multiplicité d'évidements (33) et/ou une multiplicité de zones non évidées (34).

7. Unité d'emballage (10) selon la revendication 3 ou 6, **caractérisée en ce que** le deuxième matériau d'emballage (41) fait partie du premier matériau d'emballage (31).

8. Procédé de fabrication d'une unité d'emballage (10) avec un système thérapeutique transdermique (21) contenant une couche adhésive (22),
- dans lequel au moins toute la surface (32) d'un premier matériau d'emballage (31) est dotée d'une multiplicité d'évidements (33) et d'évidements, qui sont entourés par une multiplicité de zones non évidées (34),
- dans lequel les évidements (33) sont produits de telle manière que la distance de deux évidements voisins (33) et/ou la distance de deux zones non évidées voisines (34) soit plus petite que le quintuple de l'épaisseur du matériau d'emballage et que la profondeur des évidements (33) soit au minimum de 1,2 nanomètres et au maximum de 95 % de l'épaisseur du matériau d'emballage,
- dans lequel cette surface (32) du matériau d'emballage (31) est déposée sur la face du système thérapeutique transdermique (21) disposée à l'opposé de la couche contenant un adhésif (22),
- dans lequel ce premier matériau d'emballage (31) est assemblé par soudage, pour la production de cordons de soudure (51) étanches à l'humidité, aux gaz et aux arômes, à un deuxième matériau d'emballage (41), qui est disposé sur la face du système thérapeutique transdermique (21) tournée vers la couche de principe actif et d'adhésif (22).
